# EUROPEAN PATENT SPECIFICATION

(11) **EP 0 433 260 B1**
(45) Date of publication and mention of the grant of the patent: **18.03.1998**
(21) Application number: 90870218.6
(22) Date of filing: 20.11.1990
(51) Int. Cl.: C25B 3/10, C07C 55/00, C07C 51/09

(54) **Process for preparing butanetetracarboxylate**
Verfahren zur Herstellung von Butantetracarboxylat
Procédé de préparation de butanetetracarboxylate

(30) Priority: 14.12.1989 US 450767; 14.12.1989 US 450773; 14.12.1989 US 450760
(43) Date of publication of application: 19.06.1991
(60) Divisional application: 97114709.5
(73) Proprietor: MONSANTO COMPANY, St. Louis, Missouri 63167 (US)
(72) Inventor: Casanova, Eduardo Aurelio, Ballwin, Missouri 63011 (US); Kalota, Dennis Jerome, Fenton, Missouri 63026 (US); Wagenknecht, John Henry, Cedar Hill, Missouri 63016 (US)
(74) Representative: Ernst, Hubert

(56) References cited:
- EP-A- 0 039 048
- DE-A- 1 297 091
- DE-A- 1 468 230
- DE-A- 1 944 277
- US-A- 2 264 103
- US-A- 3 193 481
- US-A- 3 193 482
- COLLECTION CZECHOSLOVAK CHEM. COMMUN., vol. 52, 1987, pages 232-2141; D. SAZOU et al.: "Electrochemical reduction of maleic and fumaric acids and their dimethyl esters in methanol at a mercury electrode"
- IDEM

## Description

### Field of the Invention

The present invention is directed to a process for preparing 1,2,3,4-butanetetracarboxylate acid by electrohydrodimerization of dialkyl maleates in alkanol to obtain tetraalkyl butanetetracarboxylates.

The compound 1,2,3,4-butanetetracarboxylic acid has been found by the U.S. Department of Agriculture to be an effective permanent press agent for polyester-cotton blend fabrics, and the compound could find use in large quantities for such purpose.
Accordingly, an efficient process for preparing the compound could be very useful. Such a process must produce a product of acceptable color performance properties, as this is an important factor for suitability for permanent press agents.

### Description of the Related Art

Procedures have been reported in which 1,2,3,4-butanetetracarboxylic acid is prepared by oxidative cleavage of tetraphthalic acid or anhydride by oxidation with ozone-containing gas, followed by molecular oxygen-containing gas, with the mixture then being heated with a peroxide, e.g. H₂O₂, at 100°C to produce the butanetetracarboxylic acid; see Japanese patent 55/49336 [80/493363], April 9, 1980, Chem. Abstracts 93 (13) 132082h; and Japanese patent 54/151906 [79/151906], Nov. 29, 1979, Chem. Abstracts, 92(23) 197937g. Also reported is a procedure in which Delta-4-tetrahydrophthalic anhydride was oxidized with HNO₃, then stirred one hour at 90°C (oxidative post treatment) to give 1,2,3,4-butanetetracarboxylic acid free of HNO₃, which gave no color on heating 30 minutes at 140°C in ethylene glycol. Polycarboxylic acids from the HNO₃ oxidation of C₅-₁₆ cycloalkenes were purified by an oxidative post treatment; see German Offen. DE 3016225 Al, Oct. 29, 1981, Chem. Abstracts, 96 (3), 19672z.

The present invention involves hydrolysis of tetraalkyl butanetetracarboxylates, which are obtained by electrolytic hydrodimerization of dialkyl maleates in alkanols.

Electrolytic reductive couplings of various activated olefins have been investigated and reported in the past. Much of this work involved aqueous systems in a divided cell, and often with a supporting electrolyte salt with a very negative discharge potential, such as a quaternary ammonium salt. In addition to reductive couplings, other reactions such as simple reduction and polymerization frequently occur. Various parameters of such reactions have been discussed, including use of various electrolytes, see Organic Electrochemistry, edited by Manuel M. Baizer and Henning Lund (1983, Marcel Dekker, N.Y., N.Y.). At page 669 of this reference, it is stated that undivided cells are operable with the restrictions that (1) the olefin and product not be substantially oxidized at the anode, and (2) the oxygen evolved at the anode in aqueous systems not promote undesirable side reactions. This reference also refers, e.g. at pages 669 and 672, to dimerization of diethyl maleate and the effect of alkali metal cations in increasing the rate of dimerization of anion radicals.

Electrolytic hyrodimerization of diethyl maleate has been reported by Baizer and Petrovich, J. Electrochem. Soc., 114(10), 1024-1025 (1967); the described procedures utilized a catholyte of water and dimethylformamide in a divided cell and indicated, all other conditions being the same, more hydrodimerization occurs in the presence of tetraethylammonium ion than of sodium ion. The electrolyses were carried out for three (3) hours, generally resulting in about 50% conversions, and specified amounts of hydrodimer, and other products.

Methanol has been used as a solvent for study of reduction mechanisms. See Dimitra Sazou et al, "Electrochemical Reduction of Maleic and Fumaric Acids and Their Dimethyl Esters in Methanol at a Mercury Electrode", Coll. Czech. Chem. Comm., 52, 2132-2141 (1957). Cyclic voltammograms of the acids in methanol solution with various supporting electrolytes, employing a hanging mercury drop electrode, are given, and reduction mechanisms discussed. The double bond reduction of the corresponding dimethyl esters was stated to take place in one step. The described procedures utilized very dilute solutions of the acids, e.g. 0.0025 or 0.005 moles per liter.

### SUMMARY OF THE INVENTION

The invention involves an efficient procedure capable of converting dialkyl maleates to tetraalkyl butanetetracarboxylates in high yields and conversions by electrolytic hydrodimerization in alkanols which can be coupled with an efficient process for hydrolyzing the tetraalkyl butanetetracarboxylates to butanetetracarboxylic acid. The electrolytic hydrodimerization provides the tetraalkyl butanetetracarboxylates in alkanol solution, a form suitable for isolution-purification by crystallization, which contributes to the purity of the butanetetracarboxylic acid produced therefrom. Some aspects of the invention relate to the acid preparation reaction, while others concern the electrolysis reaction.

The present invention involves a process for producing a tetraalkyl 1,2,3,4-butanetetracarboxylate which comprises subjecting a substantially water-free liquid electrolysis medium comprising a substantial concentration of a dialkyl maleate, an alkanol corresponding to the alkyl group of the dialkyl maleate, and an alkanol-soluble alkali metal carboxylate supporting electrolyte to electrolysis in an undivided electrolysis cell equipped with a graphite anode and a graphite cathode to effect a reductive coupling of the dialkyl maleate to yield the tetraalkyl 1,2,3,4-butanetetracarboxylate.

The present invention involves a useful preparative process for tetraalkylbutane tetracarboxylates which comprises effecting electrolytic hydrodimerization of substantial concentrations of dialkyl maleate in a medium comprising alkanol, with marked advantage in the selectivities and yields obtained, and conditions which can be employed. The invention further involves effecting such hydrodimerization in an undivided cell employing a metal salt, particularly an alkali metal salt, as supporting electrolyte. The alkanol, employed in substantially dry form, can serve as a proton donor to effect addition of hydrogen ion during the reaction. The use of alkanol, rather than water as the electrolysis medium, substantially avoids hydrolysis of the maleate ester groups, and the acidification of the medium which would result from such hydrolysis. It has been found, surprisingly, that in an alkanol medium, with an undivided cell, good yields of tetraalkylbutane-tetracarboxylate can be obtained, and that the yields in electrolyses employing sodium or other alkali metal salts can even exceed those in electrolyses employing tetraalkylammonium salts. The presence of alkanol essentially prevents hydrolysis of the dialkyl maleate, even in the presence of basic salts, as solvolysis of an alkyl group replaces it with an alkyl group. Therefore alkyl acid maleate is not formed in significant quantity, and the medium does not become strongly acidic. The hydrodimerization can therefore be carried to high conversion with good yields of hydrodimer, rather than with increasing amounts of reduction product, dialkyl succinate, resulting from acidification of the medium, as characteristic of electrolytic hydrodimerizations of dialkyl maleate in aqueous media. In aqueous media there is a shift from alkaline to acidic conditions during the electrolysis, and pH usually declines to about 4. In the absence of water in an alkanol medium, such acidic pH conditions do not develop and a marked increase in succinate product is not observed. The present process is marked by an absence of substantial amounts of monoalkyl maleate in the electrolysis medium. It is very advantageous that the present process can be conducted efficiently in an undivided cell, thereby avoiding the additional electrical resistance, membrane expense, and other adverse factors involved in operating with a divided cell. The invention generally involves use of electrolysis solution with very substantial concentrations of maleate reactant and use of fairly substantial electrical current in the electrolysis, and obtaining substantial amounts of tetraalkyl butane tetracarboxylate product in reasonable reaction time.

### BRIEF DESCRIPTION OF THE DRAWINGS

Fig. 1 is a drawing with bar graphs illustrating yields of tetramethyl butanetetracarboxylate and other products obtained by electrolysis in methanol with various electrolytes.
Fig. 2 is a drawing with bar graphs illustrating yields of tetramethyl butanetetracarboxylate and other products obtained by electrolysis in methanol employing various cathodes.

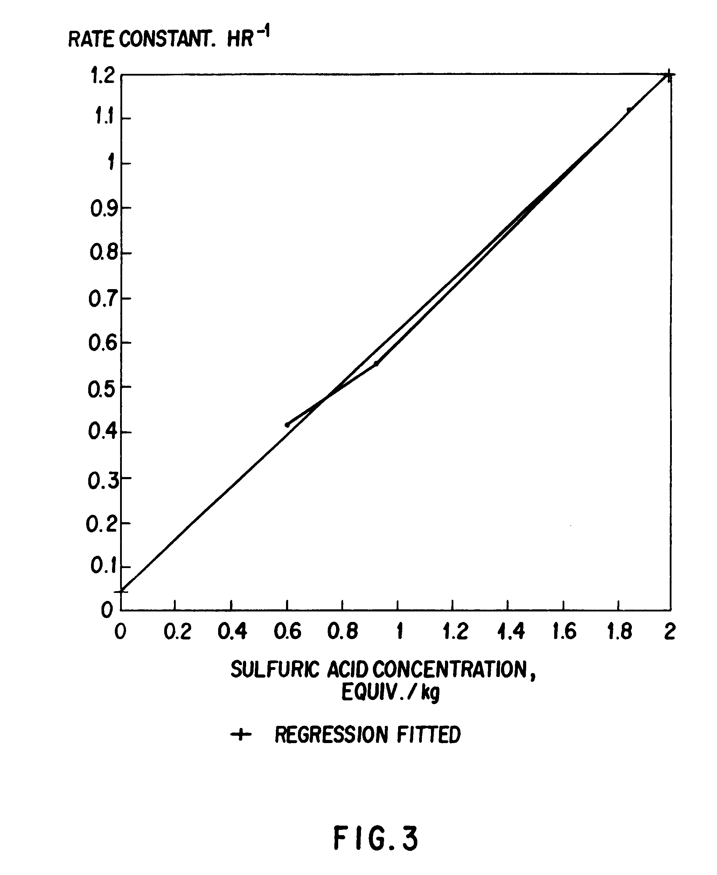

Fig. 3 is a graph of TMBTC hydrolysis rate constant vs. acid concentration.

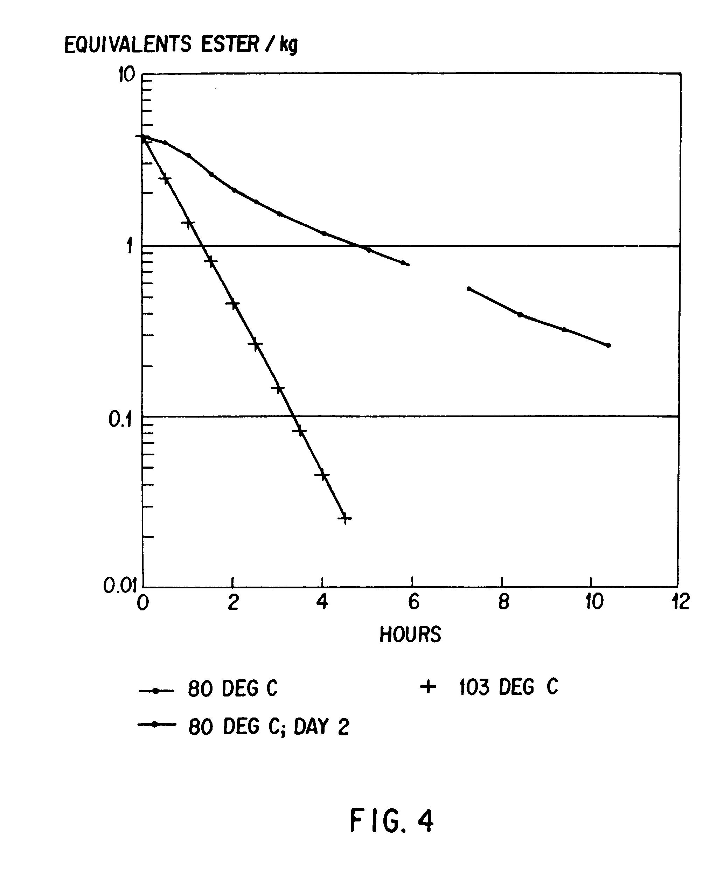

Fig. 4 is a graph showing TMBTC hydrolysis vs. time at different temperatures.

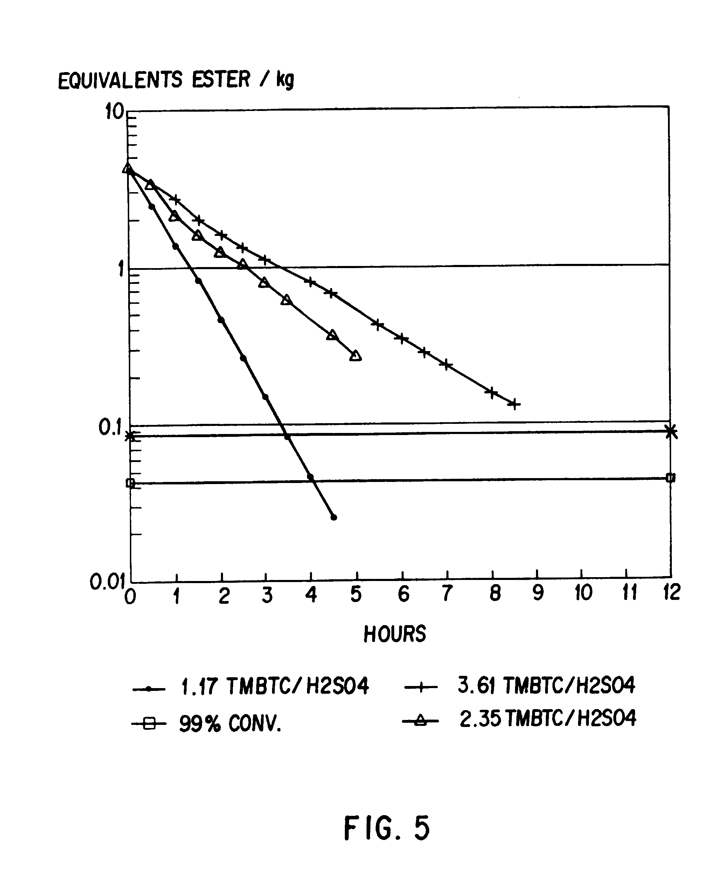

Fig. 5 is a graph showing TMBTC hydrolysis vs. time for different TMBTC/H₂SO₄ mole ratios.

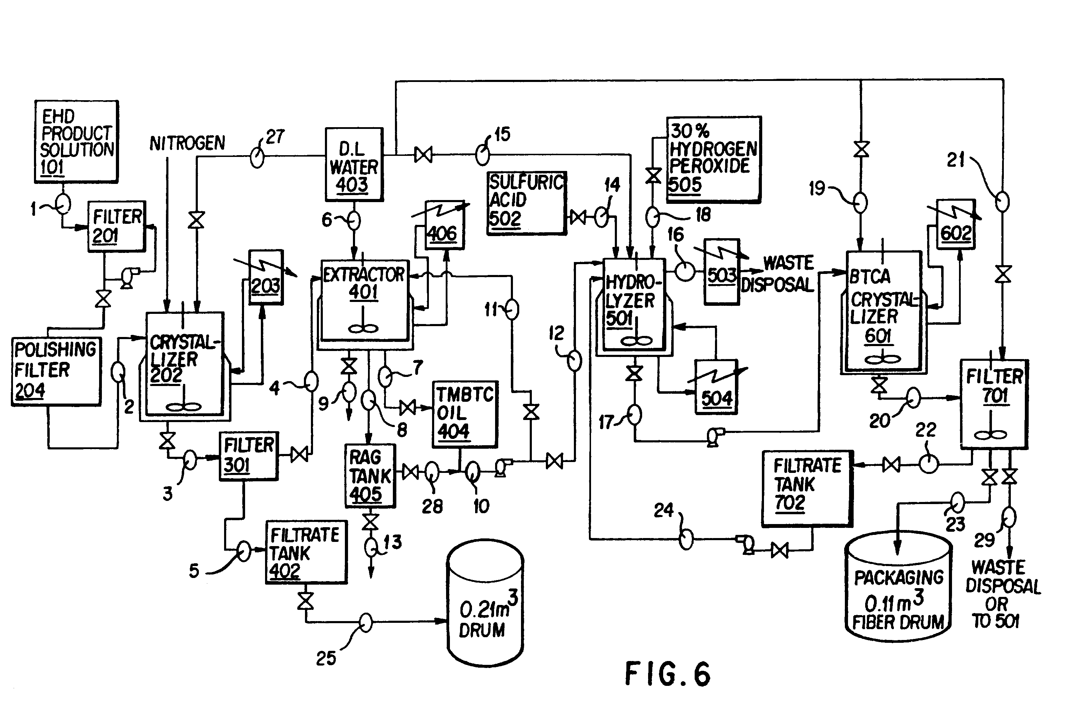

Fig. 6 is a flow sheet for an exemplary process for preparing BTCA.

### DESCRIPTION OF THE PREFERRED EMBODIMENTS

In accordance with the present invention, a process is provided for the preparation of 1,2,3,4-butanetetracarboxylates.

### Electrolytic Hydrodimerization

The electrolytic hydrodimerization step of present process can be conducted with dialkyl maleates in general, but for practical considerations, only the maleates with lower alkyl groups, e.g. of 1 to 6 carbon atoms, are likely to be of much interest. Dimethyl maleate (DMM) is the preferred reactant, and is used in exemplifications herein, but diethyl maleate, dipropyl maleate, dihexyl maleate, etc. can be used. Electrical resistance tends to increase with increasing alkyl size, whether in the ester or in the alkanol solvent, thereby making electrical power usage less efficient. It is also disadvantageous to employ alkanols of such high molecular weight that they tend to be solids at ambient temperature.

The reactions presumably occurring in the electrolysis of the present process can be pictured: 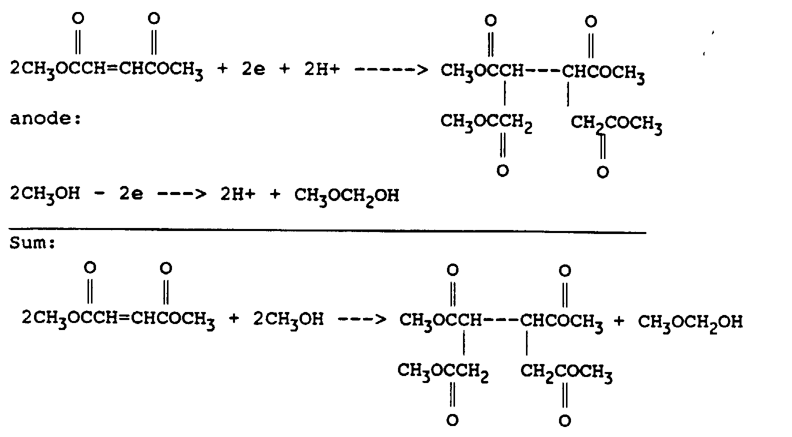 Methoxymethanol, the hemiacetal of formaldehyde, is also a likely product. The presence of formaldehyde has been confirmed, however, its presence may be due to disassociation of methoxymethanol. Additional possible intermediates include +CH₂OH and •CH₂OH in the anode reaction, and acetic acid from protons and acetate electrolyte (if used). Also, alkoxides, e.g. CH₃O⁻, can be produced as a result of reaction at the cathode.

With •CH₂OH as a likely intermediate at the anode, it presents the possibility of adding at the maleate double bond to cause production of other by-product, thereby possibly causing considerable loss in selectivity to the desired hydrodimer when an undivided cell is used; however, this undesirable side reaction does not appear to occur to any significant extent as good results are obtained in an undivided cell. It may be that the use of an undivided cell is actually advantageous, as it permits protons generated at the anode to move very freely to protonate methoxide produced in conjunction with the hydrodimerization at the cathode, thereby avoiding possible interfering reactions of the methoxide and polymerization.

It has fortunately been found that the present electrolysis can not only be carried out very efficiently with simple alkali metal salts as supporting electrolyte, but that the results with such salts are actually better than those obtained with some of the more expensive electrolytes which are commonly used. In addition to the preferred alkali metal salts, the present process can use other supporting electrolytes known to the art.

The electrolysis uses supporting electrolytes to provide ions to carry the current in the process. In general, any electrolytes can be employed which dissociate into ions in the electrolysis medium to carry current, and which do not unduly interfere with the desired reactions or cause excessive losses to competing reactions. Most of the electrolytes can be considered salts, as having a cation from a base and an anion from an acid. However, it is also feasible to employ bases as the electrolytes, and this may at times be appropriate in order to counter acidity. The dialkyl maleates are subject to reduction at less negative potentials than many suitable cations, so competitive discharge of cations is not ordinarily a concern. Alkali metal compounds such as sodium, potassium or lithium compounds, can be employed, as well as alkaline earth metal compounds, and quaternary ammonium compounds, which are characterized by very negative discharge potentials. Acid anions will in general be operative as the anionic portion of the electrolyte, but will generally be selected to have acceptable solubility in the alkanol system, and to minimize interfering or competing reactions and electrode degradation. Among operable anions are carboxylic acid anions, halide ions, and aromatic sulfonic acid anions.

In the present invention it has fortunately been found that a very simple salt, e.g. sodium acetate, serves very well as an electrolyte in the electrohydrodimerization of alkyl maleates. In the prior art quaternary ammonium salts have usually been considered to give better results in electrohydrodimerization than do alkali metal salts. However, in the present invention alkali metal salts have been found capable of giving better results in the electrohydrodimerization of dimethyl maleate, particularly with respect to selectivity to desired product. Among the useful electrolytes are sodium, potassium and lithium acetates, propionates, and succinates, sodium toluenesulfonates, tetrabutylammonium p-toluenesulfonate, tetrabutylammonium hydroxide, tetrabutylammonium acetate, tetrabutylammonium chloride. Similar salts can be used with sulfate, phosphate and tetrafluoroborate anions, but such salts tend to cause anode degradation when preferred graphite anodes are used. Some halide salts, e.g. sodium halide, have very limited solubility in methanol, and are therefore inconvenient for use. With regard to calcium chloride, the chloride is theorized to be relatively tightly bound to the calcium and to act as an acid catalyst to cause formation of dialkyl 2-methoxysuccinate (conveniently referred to herein as methoxydialkylsuccinate), making selectivity very poor to the desired hydrodimer. Calcium acetate has poor solubility, but calcium nitrate is better in this regard.

The present electrolysis process can be carried out over a broad range of electrolytic conditions, including a wide range of strengths of applied electric currents and current densities at the electrodes. The process is operable at very low current densities, such as less than 5 milliamperes per square centimeter to more than 100 or 200 milliamperes per square centimeter. Preferred current densities are apt to be at least 15 : more preferably in the range of 15 to a 50 or so milliamperes per square centimeter, with operation, for example at 25 milliamperes per square centimeter giving good product selectivity at relatively low cell voltage, with good electrode life. There is advantage in having high current density in order to maximize cell utilization, but this is to be balanced against the high cell voltage and resistance and heat generation which add to costs.

The present electrolysis can be operated over a broad range of concentrations, such as from less than 5% to more than 50% by weight of the dialkyl maleate reactant, and good selectivities to the desired dimer products are obtainable over broad ranges. Concentrations from at least 15% to 35% to 40% or so are usually very suitable, and product concentrations in the same range are also very suitable, although they will be lower in specific cases because of less than 100% yields and conversions. The process is suitable for large scale production, making kilogram or more quantities of product. The use of relatively high concentrations of reactant lessens the amount of materials to be handled. However, the electrical resistance of the solution rises with the concentration of reactant. In addition, solubility considerations may be a factor at some higher concentrations. It is desirable, although not necessary, to operate with all components in a homogeneous phase during the electrolysis.

The concentration of supporting electrolyte can vary widely, but it is unnecessary to have more than very dilute concentrations for conductivity. Higher concentrations will improve conductivity, but salts in general are not very soluble in methanol, and there is ordinarily no advantage in using amounts of salts in excess of their solubility. The amount of salt can be just a minimum amount to give electrical conductivity, but will generally be in a range of 0.5 to 2 or 3% or so by weight, and for practical purposes, seldom over 5% or so by weight. In order to minimize expense, the salt concentrations will be kept low, as the cost of replenishing or recycling the salts will increase with the amount of the salt. The preferred operation will employ a relatively inexpensive salt, e.g. sodium acetate, which can be disposed of, rather than recycled.

The concentration ranges of maleate reactant set forth herein are, in general, initial concentrations, as the concentration will change during the electrolysis process, which will generally be run as a batch reaction, or a series of batch reactions. The electrolysis reaction will ordinarily be run to fairly high conversion, reacting more than 75% or 80% of the maleate, because selectivity to desired product is still good at high conversions, and in order to avoid unnecessary steps, handling and expense in separating unreacted maleate from the dimer product for recycle. It will be preferred to operate at maleate conversions approximately 95% or so. Higher conversions are possible, but it has been found that significant electrode degradation occurs if the electrolysis is continued with little or no maleate reactant present.

It has been found that there is a competing chemical side reaction which produces methoxydimethylsuccinate. The amount of this reaction is generally dependent upon the time of exposure of the maleate reactant to the components of the reaction system. Therefore it may be desirable to operate the electrolysis as a series of batch reactions, with relatively low initial maleate concentration and addition of more maleate in subsequent batches of the series. The last batch could then be taken to high conversion prior to product separation. Another approach to minimizing maleate contact time is to use an electrolysis cell which is large, particularly with respect to electrode surface area, compared to the amount of material in the reaction system and maleate reactant. Another approach is a constant stirred tank reactor with a continuous feed and discharge where the DMM concentration is maintained low to diminish the chemical driving force for this side reaction.

The control of electrolytic reaction time can also be expressed in terms of electrical current supply. The conversion of a particular amount of maleate reactant requires a corresponding number of ampere-hours of current, and the time to accumulate a requisite number of ampere-hours in an electrolysis can be varied by changing the current, or the number, or size of electrolysis cells. A reaction in accord with descriptions herein within 15 hours is fairly efficient, but a reaction time of no more than 10 hours will give less by-product. If the same current is involved, a 16-cell aggregate as described herein will accumulate ampere hours at twice the rate of an 8-cell aggregate. Of course, the 16 cells also use higher voltage for equivalent current. Cells for large scale production are contemplated as using at least 5 amperes, and more likely 10 or more amperes. Taking into consideration the amperage and number of cells employed, the present process will ordinarily use current and maleate amounts such that no more than 100 grams of dimethyl maleate are present per cell-ampere, and preferably less than 50 grams, or possibly even less than 25 grams dimethyl meleate per cell-ampere. (The term cell-ampere is number of cells x amperes, and is equivalent to ampere-hours per hour).

The present electrolysis can be effected with the usual electrodes employed in electrohydrodimerization and other reductive coupling reactions. Various metal and graphite electrodes are suitable. The preferred electrodes will generally have relatively high hydrogen overvoltages, such as greater than that of copper. However, lower overvoltage electrodes can be used. Among the cathode materials which can be used are graphite, graphite felt, mercury, copper amalgam, gold, copper, cadmium, tin and aluminum, with graphite, graphite felt, and lead being among the better materials. Mercury is an effective cathode, but its liquid state makes it unsuitable for common flow cell configuration. Graphite electrodes, whether plate or felt, have been found to give the best results. It is an advantage of the present process, and surprising, that it can be conducted with superior results at graphite electrodes. Graphite is much less expensive than many other electrode materials, such as platinum or even lead or cadmium electrodes, does not add heavy metals to the solution via corrosion and is suitable for anodes as well as cathodes.

The present electrolysis can be carried out well with an alkanol, e.g. methanol, as the only material employed as carrier for the maleate ester and electrolyte salt. Ordinary industrial grades of methanol, which are substantially water-free, are very suitable for use. Traces of water picked up from contact with the atmosphere will not ordinarily be sufficient to affect results. For example, 2000 ppm water in solution has negligible effect. However, presence of more than traces of water will preferably be avoided, as even a small percentage of water can cause a decline in selectivity, and presence of more than, say 5% by weight, of water is very undesirable. If desired, co-solvents can be used along with the alkanol, particularly such aprotic solvents as dimethyl formamide and dimethyl sulfoxide or acetonitrile. Use of co-solvents will not generally be desirable, but there may be particular cases where solubility or other factors would make co-solvents worthwhile.

At the end of the electrolysis reaction the tetraalkyl butanetetracarboxylate product is present in solution in the electrolysis medium, for example, at a concentration of about 25% by weight. The tetraalkyl butanetetracarboxylate product can be separated by crystallization from the solution, followed by filtration. In the case of tetramethyl butanetetracarboxylate (TMBTC), the crystallization is effected by cooling, e.g. to below 0°C, usually between 0°C and -10°C. The separation removes the product from the electrolysis medium and also separates it from residual maleate reactant and succinate and alkoxysuccinate by-products. The butanetetracarboxylate tetraester product can then be subjected to hydrolysis and purification procedures to prepare butanetetracarboxylic acid suitable for permanent press use.

The invention is illustrated by the following examples.

### Electrolytic Hydrodimerization

### EXAMPLE 1

An electrolysis was carried out in a jacketed resin pot, using water as an electrolysis medium, with dimethyl maleate present as a second phase, constituting 22% by weight of the electrolysis medium. The cathode was lead, and the anode was platinum. A mixture of tetrabutylammonium nitrate and tetrabutylammonium hydroxide was employed as electrolyte, and electrolysis was conducted at a current density of 30 milliamperes per square centimeter of cathode surface. The electrolysis began at a basic pH, but rapidly became more acidic due to base catalyzed hydrolysis of dimethyl maleate, leading to monomethyl maleate. The pH quickly approached a value of 4. Analysis showed a weight ratio of 47 parts tetramethyl butanetetracarboxylate to 22 parts of dimethyl succinate, a simple reduction product of the starting maleate. This amounts to a selectivity of only 2.1 parts hydrodimer to 1 part of the succinate material. It is apparent that the acidic conditions are causing a large loss to a simple reduction reaction, and that even the use of a basic electrolyte did not prevent the development of acidic conditions. The analysis of the electrolysis medium also showed unreacted dimethyl maleate, with it being present in a ratio of 41 parts to the 47 and 22 parts of hydrodimer and succinate products. Thus the reaction had been taken to only a relatively low conversion. Similar results were obtained in other runs with aqueous media, employing the undivided resin pot cell and graphite or lead cathodes with platinum anodes, at current densities varying from 30 to 70 milliamperes per square centimeter. Electrolytes utilized included tetrabutylammonium nitrate, tetraethylammonium p-toluenesulfonate, tetrabutylammonium hydroxide and tetrabutylammonium sulfate. The ratio of hydrodimer to succinate varied from the 2.1 reported above, to 0.43, with higher values being obtained when excess tetrabutylammonium hydroxide was present in an attempt to maintain a high pH.

### EXAMPLE 2

Electrolyses were carried out utilizing an undivided resin pot cell as described in Example 1, but using methanol as the medium. Results for a number of electrolyses, with quaternary ammonium electrolytes with some variation in conditions and electrodes, are set forth in Table 1. In the Table, the numerical values for dimethyl maleate (DMM) dimethyl succinate (DMS), and tetramethyl butanetetracarboxylate (TMBTC) are reported in terms of analytical values, which can be compared to give the ratios of the reported materials. The ratio of TMBTC to DMS ranged from as high as 2.55 in Run 1, down to 0.89 in Run 3, with the results in general being better than those with water as solvent. The Runs 5 and 6 used, respectively, 90% methanol and 33% methanol, with the results being inferior to those obtainable with undiluted methanol.

**TABLE 1**

| Run | Elec | Cath | Anode | Pl | Temp | CD | DMM | DMS | MeODMS | TMBTC |
|---|---|---|---|---|---|---|---|---|---|---|
| 1 | TBAFB | Gr | Pt | 26 | 25 | 70 | 68 | 29 | | 74 |
| 2 | TBAFB | Hg | Pt | 26 | | 40 | 5 | 18 | 35 | 45 |
| 3 | TBAFB | Gr | Pt | 26 | | 70 | 9 | 45 | | 40 |
| 4 | TBAH | Gr | Gr | 26 | | 70 | 0 | 39 | | 42 |
| 5x | TBAFB | Gr | Pt | 25 | | 70 | 20 | 30 | | 55 |
| 6xx | TEAT | Pb | Pt | 15 | | 30 | 31 | 35 | | 41 |
| x 90% methanol in water | | | | | | | | | | |
| xx 33% methanol in water | | | | | | | | | | |

In Table 1 and elsewhere in the specification abbreviations will at times be used as designations as follows:
DMM is dimethyl maleate;
DMS is dimethyl succinate;
MeODMS is methoxydimethyl succinate;
TMBTC is tetramethylbutane tetracarboxylate;
TBAFB is tetrabutylammonium tetrafluoroborate;
TBAH is tetrabutylammonium hydroxide;
TEAT is tetraethylammonium-p-toluenesulfonate;
Pl is the payload in % by weight DMM in solution; and
CD is current density in milliamperes/cm²

### EXAMPLE 3

The undivided resin pot cell of Example 1 was utilized with methanol as the medium and small concentrations of metal salts as electrolyte, with results reported in Table 2.

**TABLE 2**

| | | | | Pl | Temp | CD | | | | |
|---|---|---|---|---|---|---|---|---|---|---|
| Run | Elec | Cath | Anode | (%) | (°C) | (mA/cm²) | DMM | DMS | MeODMS | TMBTC |
| 1 | NaOAc | Felt | Gr | 25 | 15 | 50 | 0 | 12 | 2 | 86 |
| 2 | Na₂Suc | Gr | Gr | 25 | 15 | 50 | 0 | 22 | 32 | 55 |
| 3 | KOAc | Gr | Gr | 26 | 27 | 70 | 1.7 | 8.2 | 1.8 | 15.6 |
| 4 | LiOAc | Gr | Gr | 26 | 28 | 70 | 2.5 | 4.5 | 1.5 | 16.2 |

It is demonstrated that good selectivities can be obtained by employing alkali metal salts in methanol, as seen from the 7.17 hydrodimer to succinate ratio (86/12) in Run 1, with sodium acetate and high conversions were also obtained as shown by the low or zero values for dimethyl maleate in the product solution.

### EXAMPLE 4

Electrolyses were conducted in a small flow cell of parallel plate design with a gap between electrodes of about 1 mm, and cathodes of 19 cm₂. Flow through the cell was at about 1 liter/minute. The cell was connected to a jacketed reservoir which was cooled by tap water (at about 15°C). Electrolyses were conducted with dimethyl maleate, and about 1% by weight of a selected metal salt, in methanol, with results as reported in Table 3.

**TABLE 3**

| | | | | Pl | Temp | CD | | | | |
|---|---|---|---|---|---|---|---|---|---|---|
| Run | Elec | Cath | Anode | (%) | (°C) | (mA/cm²) | DMM | DMS | MeODMS | TMBTC |
| 1 | NaOAc | Gr | Gr | 44 | 25 | 50 | 15 | 20 | 6 | 71 |
| 2 | LiOAc | Gr | Gr | 42 | 27 | 50 | 47 | 13 | 7 | 37 |
| 3 | LiOAc | Gr | Gr | 26 | 27 | 50 | 9 | 34 | 3 | 59 |

While there was considerable variation in these and other results in the cell, ratios of dimer to succinate as high as 3.5 were obtainable employing alkali metal salts in methanol.

### EXAMPLE 5

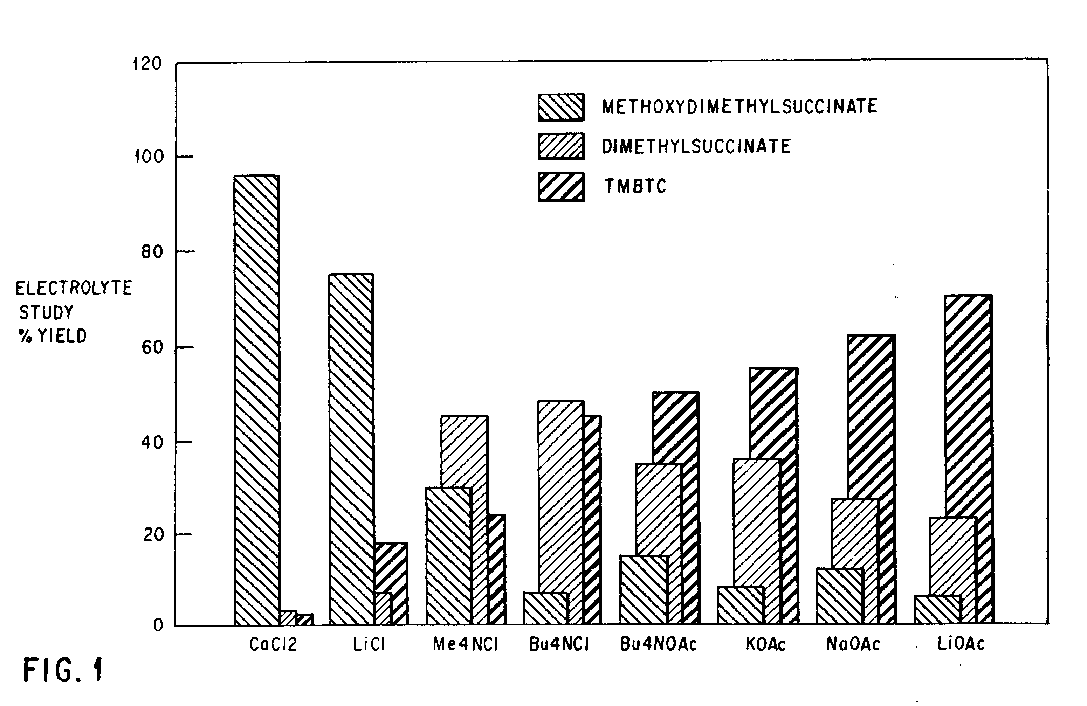

Electrolyses of dimethyl maleate were carried out in methanol employing various electrolytes. The electrolysis cell was a jacketed resin pot of 150 ml capacity, fitted with a magnetic stirring bar, graphite plate electrodes (5 cm by 5 cm by 0.5 cm) with 25 cm₂. of cathode surface facing the anode. The cell was cooled with tap water (15 to 20°C) Power was supplied by a constant current power supply, generally set at 1 ampere. The cell was charged with 75 g methanol, 25 g dimethyl maleate, and 1 to 2 g of supporting electrolyte. Electrolysis was started and continued until nearly all of the dimethyl maleate was consumed as determined by gas chromatography. Selectivity to the three major products (as a percentage of the three products) was determined by gas chromatography, and shown in the bar graphs of Fig. 1. It will be noted that high TMBTC selectivities are obtained with the alkali metal acetates, particularly with lithium and sodium acetates. The illustrated results are based on generally comparable procedures. While other results may be obtained under other conditions, the illustrated results show that high selectivities are obtainable, and this is consistent with the selectivities consistently obtainable, particularly with sodium acetate, under standard conditions in other procedures. Halide anions were operable, although at a very low selectivity with CaCl₂. The high levels of methoxydimethylsuccinate indicate that use of CaCl₂ and LiCl results in catalytic methoxylation, a competing reaction.

### EXAMPLE 6

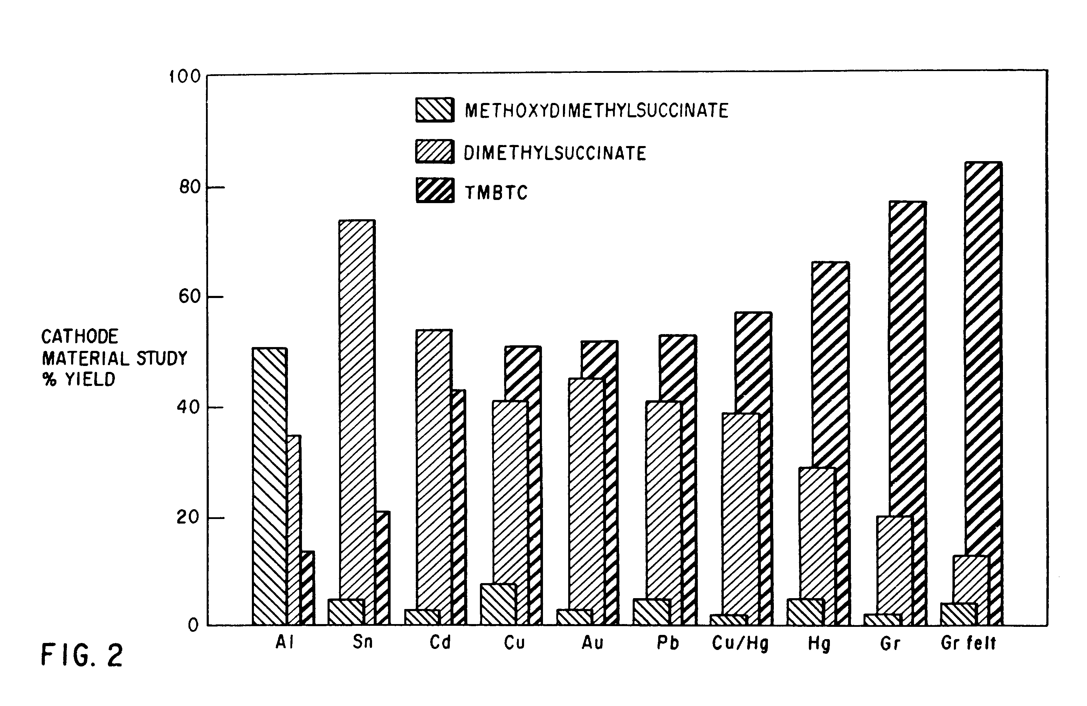

Electrolysis was conducted as in Example 5, with sodium acetate as electrolyte, and employing various cathodes. The selectivities obtained for the three major products are illustrated in the bar graphs of Fig. 2.

### EXAMPLE 7

A large flow cell was utilized to prepare tetramethyl butanetetracarboxylate in an electrolysis with sodium acetate as electrolyte. The cell was a modified Electro Syn Cell, (Svenska Utveklingsaketbologet, Swedish National Development Company) with 8 cells, later modified to 16 cells. The cell had 500 cm² graphite plates with about 1 mm spacing and plastic screens between electrodes to aid in flow dispersion. The cell was attached by polyvinyl chloride piping to a centrifugal pump, 18.93 liter (5 gallon) reservoir and stainless steel heat exchanger. The system was charged with about 8 kg dimethyl maleate, 15 kg of methanol and 200 g sodium acetate. The solution was circulated through the cell at about 75.7 liters (20.0 gallons) per minute. The cell was operated at 12.5 amperes (65-90 volts) for about 7.5 hours (with 16 cells). Typical analysis of the resulting solution was 25% tetramethyl butanetetracarboxylate, 5% dimethyl succinate, 5% methoxydimethylsuccinate, 5% dimethyl maleate, and the balance methanol.

To describe in more detail, the cell had been modified to operate in a bipolar mode with only the end plates attached to the electrical supply. Stated quantities (reported in Table 4 below) of dimethyl maleate, methanol and sodium acetate were charged into the reservoirs as listed under DMM, MeOH and NaOAc. The circulation pump was activated and circulation was effected to achieve sample homogeneity, at a flow rate of 75.7 liters-79.5 liters (20-21 gallons) per minute. A sample was drawn at time zero, with DMM usually below charge quantity because of dilution by residue of a previous run. The power supply was activated and electrolysis conducted at 25 mA/cm² until the power was shut off at reported times. A sample was drawn and analytical results reported as %'s of reaction mixture, along with the selectivities to the products determined therefrom, and the grams of DMM which had reacted. Results of three different runs are reported in Table 4. Product selectivity as high as 83% was obtained in the third run, and thus, like the other runs in methanol, was carried to a high conversion, the conversion of dimethyl maleate being over 95%, based on the maleate in the product sample analysis, and the amount of reacted maleate. A comparison of Run 1 with an 8 cell electrolysis, to Runs 2 and 3 with 16 cell electrolysis, shows that increasing the cells can cut the reaction time and also cut the amount of methoxydimethyl succinate product, thereby improving selectivity to the desired hydrodimer. In general the production of the by-product, produced by a chemical reaction, can be lessened by operating with a high electrolysis cell through-put compared to reservoir capacity, or other means to cut reaction time, as well as by limiting payload or lowering reaction temperature.

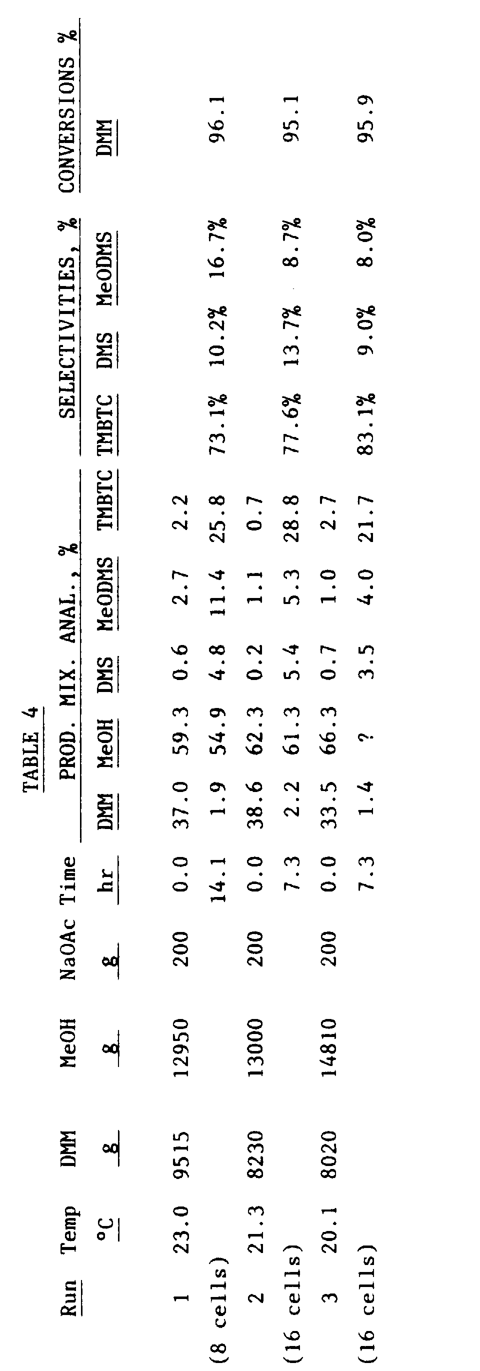

### EXAMPLE 8

Tests were conducted to determine the effect of temperature on the degree of recovery of tetramethyl butanetetracarboxylate from methanol, and the solubility of the compound in methanol at temperatures in the range of interest. The starting concentration was about 25% of the TMBTC compound. Results are reported in Table 10.

**TABLE 5**

| Temp.(°C) | Recovery (%) | Solubility (%) |
|---|---|---|
| -11 | 87.8 | 5.07 |
| - 7 | 83.5 | 6.49 |
| - 6 | 82.2 | 7.32 |
| - 1 | 78.6 | 9.22 |

### EXAMPLE 9

A methanol solution containing TMBTC and various impuruties was separated by crystallization and filtration into 33% crystals and 67% filtrate, and partition of the various components between crystals and filtrate was determined at -10°c temperature with results reported in Table 11.

**TABLE 6**

| | MeOH(%) | DMM(%) | DMS(%) | MeO-DMS(%) | TMBTC(%) |
|---|---|---|---|---|---|
| Crystals | 2.7 | 0 | 7.4 | 0 | 83.2 |
| Filtrate | 97.3 | 100 | 92.6 | 100 | 16.8 |

From the results in Table 10 it is evident that lower temperatures markedly improve TMBTC recovery, with -11°C., the lowest temperature shown, giving the best results. The results in Table 10 show that the crystallization is an effective means to separate TMBTC from various impurities, as well as from the methanol solvent.

### EXAMPLE 10

The effect of water on the recovery of TMBTC from methanol solution was tested, employing about a 25% TMBTC concentration and a -10°C. crystallization temperature. Results are reported in Table 12.

**TABLE 7**

| % Water | % TMBTC Recovered |
|---|---|
| 0 | 88.1 |
| 5 | 89.9 |
| 10 | 90.6 |
| 20 | 92.9 |
| 40 | 94.1 |
| 75 | 97.6 |

The percentages of water are based on the total solution, i.e. 75% water means a solution with 75% water content. It is evident that the recovery is improved by increasing the water content. Of course, additional water utilizes space in the crystallizer, thereby lessening the payload of TMBTC.

The use of water in the crystallization medium can improve the separation from dimethyl succinate, although this will vary considerably with the percentages of water employed. Table 13 shows the variance in TMBTC composition with % water content.

**TABLE 8**

| COMPOSITION OF TMBTC | | | | |
|---|---|---|---|---|
| Water (%) | TMBTC (%) | DMS (%) | CH₃OH (%) | Water (%) |
| 0 | 88.1 | 10.6 | 6.0 | 0.3 |
| 5 | 77.8 | 12.7 | 8.0 | 1.5 |
| 10 | 75.3 | 13.1 | 8.9 | 2.7 |
| 20 | 75.2 | 10.8 | 8.3 | 5.7 |
| 40 | 75.0 | 9.2 | 5.8 | 10.0 |
| 75 | 73.5 | 5.6 | 2.6 | 18.3 |

Filtrations of the TMBTC solution have been found very useful for their effect upon later extraction procedures, particularly when the solutions were obtained by EHD reactions. The filtrations are employed to filter out insoluble impurities from the TMBTC solutions. In a particular case, an unfiltered EHD solution took up about 1/3 of the volume of an extractor with a rag layer, which resisted separation. With a filtered EHD solution, the rag layer was only about 5% of mass.

The starting TMBTC solution utilized herein, as obtained by an EHD reaction of dimethyl maleate, is characterized by the presence of small amounts of particular reactants, by-products and other impurities. Among those materials included are dimethyl maleate, dimethyl succinate, and methoxydimethylsuccinate. These materials are separated fairly effectively in a crystallization and filtration step, as the materials largely remain in the methanol and go to filtrate, while the TMBTC is filtered out as crystals.

Water extractions, as used in the processing, are useful for removing electrolyte salt and some color materials. Some methanol is also removed, but this has little significance as methanol is produced and removed downstream in the hydrolysis stage. A TMBTC solution, as provided from an EHD reaction, has a yellow color. This can be from corrosion of connections, e.g. titanium connections, on EHD electrodes, and from organic color bodies. The water extractions mostly remove the color from the titanium, and partially remove that from organic contaminants. A second extraction appears to remove color beyond that removed by the first extraction. However, the number of extractions to be used will depend upon the degree of contamination, as well as the time and efficiency of the extraction procedure. Also the extractions can be tailored to that which is appropriate in conjunction with a later oxidation treatment to have a sufficient removal of color or color-forming materials. The extractions also remove salts, e.g. sodium acetate. The water extractions can very suitably be performed with the tetramethyl butanetetracarboxylate being the material purified, as this ester has very limited water solubility. In contrast, the downstream hydrolysis product, butanetetracarboxylic acid, has a fair degree of water solubility and would not lend itself to efficient extraction. The term "extraction" is used herein in the sense that the TMBTC is washed with water to extract impurities therefrom, while the TMBTC itself is not dissolved in the aqueous system. For the extractions, any effective way of mixing the TMBTC with an aqueous system, following by separation can be used. Rather than the batch system illustrated herein, a counter-current system could be employed in which streams are mixed and then separated.

There are various possible approaches and routes to preparation of butanetetracarboxylic acid which do not involve tetraalkyl butanetetracarboxylates. From theoretical considerations, tetraalkyl butanetetracarboxylates might be expected to be difficult to hydrolyze, as involving four electron-withdrawing groups on adjacent carbon atoms. However, it has been found feasible to hydrolyze tetraalkyl butanetetracarboxylates to virtually 100% completion, hydrolyzing all four ester groups, in reasonable reaction times and with nearly quantitative yields; and to conduct an overall process with various purification procedures, starting with a tetraalkyl butanetetracarboxylate still in its preparative reaction mixture, as e.g. an EHD electrolysis solution obtained using procedures in accordance with the present invention, and obtain butanetetracarboxylic acid of acceptable purity in overall yield of 80-85%.

## Claims

1. A process for producing a tetraalkyl 1,2,3,4-butanetetracarboxylate which comprises subjecting a substantially water-free liquid electrolysis medium comprising a substantial concentration of a dialkyl maleate, an alkanol corresponding to the alkyl group of the dialkyl maleate, and an alkanol-soluble alkali metal carboxylate supporting electrolyte to electrolysis in an undivided electrolysis cell equipped with a graphite anode and a graphite cathode to effect a reductive coupling of the dialkyl maleate to yield the tetraalkyl 1,2,3,4-butanetetracarboxylate.

2. The process of Claim 1 in which the dialkyl maleate is dimethyl maleate, the alkanol is methanol, and the tetraalkyl 1,2,3,4-butanetetracarboxylate is tetramethyl 1,2,3,4-butanetetracarboxylate.

3. The process of Claim 1 in which the electrolysis is conducted at a rate sufficient to keep the amount of dialkyl 2-methoxy succinate produced in a competing chemical reaction to a value less than 5% of the dialkyl maleate reacted.

4. The process of Claim 1 in which the alkanol-soluble alkali metal carboxylate supporting electrolyte is sodium acetate.

5. The process of Claim 2 in which the tetramethyl 1,2,3,4-butanetetracarboxylate is separated from the electrolysis medium by cooling and crystallization.

6. The process of Claim 2 in which the concentration of dimethyl maleate initially in the electrolysis medium is at last 15% by weight and the electrolysis is continued until at least about 95% of the dimethyl maleate has reacted.

7. The process of Claim 6 in which the electrolysis cell is a flow cell which is used at current densities of at least 15 mA/cm².

8. The process of Claim 7 in which the current densities are in the range of 15 to 100 mA/cm².

## Patentansprüche

1. Verfahren zur Herstellung von Tetraalkyl-1,2,3,4-butantetracarboxylat, welches die Unterwerfung eines im wesentlichen wasserfreien flüssigen Elektrolysemediums, das eine erhebliche Konzentration eines Dialkylmaleats, eines Alkanols, das der Alkylgruppe des Dialkylmaleats entspricht, und eines Eletrolyten zur Unterstützung des Alkanol-löslichen Alkalimetallcarboxylats einer Elektrolyse in einer ungeteilten Elektrolysezelle, die mit einer Graphitanode und einer Graphitkathode ausgestattet ist, um eine reduktive Koppelung des Dialkylmaleats herbeizuführen, umfaßt, um das Tetraalkyl-1,2,3,4-butantetracarboxylat zu erhalten.

2. Verfahren gemäß Anspruch 1, wobei das Dialkylmaleat Dimethylmaleat ist, das Alkanol Methanol ist, und das Tetraalkyl-1,2,3,4-butantetracarboxylat Tetramethyl-1,2,3,4-butantetracarboxylat ist.

3. Verfahren gemäß Anspruch 1, wobei die Elektrolyse mit einer Geschwindigkeit durchgeführt wird, die ausreicht, um die Menge Dialkyl-2-methoxysuccinat, die in einer konkurrierenden chemischen Reaktion erzeugt wird, bei einem Wert von weniger als 5% des umgesetzten Dialkylmaleats zu halten.

4. Verfahren gemäß Anspruch 1, wobei der Elektrolyt zur Unterstützung des Alkanol-löslichen Alkalimetallcarboxylats Natriumacetat ist.

5. Verfahren gemäß Anspruch 2, wobei das Tetramethyl-1,2,3,4-butantetracarboxylat durch Abkühlung und Kristallisation vom Elektrolysemedium abgetrennt wird.

6. Verfahren gemäß Anspruch 2, wobei die Konzentration von Dimethylmaleat im Elektrolysemedium am Anfang mindestens 15 Gew.% beträgt und die Elektrolyse fortgesetzt wird, bis mindestens 95% des Dimethylmaleats umgesetzt sind.

7. Verfahren gemäß Anspruch 6, wobei die Elektrolysezelle eine Fließzelle ist, die bei Stromdichten von mindestens 15 mA/cm² verwendet wird.

8. Verfahren gemäß Anspruch 7, wobei die Stromdichten im Bereich von 15 bis 100 mA/cm² liegen.

## Revendications

1. Procédé de préparation de 1,2,3,4-butane-tétracarboxylates de tétraalkyle, qui comporte le fait de soumettre à une électrolyse un milieu d'électrolyse, liquide et pratiquement anhydre, contenant un maléate de dialkyle en une concentration importante, un alcanol correspondant au groupe alkyle du maléate de dialkyle, et comme électrolyte support, un carboxylate de métal alcalin, soluble dans l'alcanol, ce milieu étant placé dans une cellule d'électrolyse non divisée et munie d'une anode en graphite et d'une cathode en graphite, afin de réaliser le couplage réducteur du maléate de dialkyle pour obtenir un 1,2,3,4-butane-tétracarboxylate de tétraalkyle.

2. Procédé conforme à la revendication 1, dans lequel le maléate de dialkyle est du maléate de diméthyle, l'alcanol est du méthanol, et le 1,2,3,4-butane-tétracarboxylate de tétraalkyle est du 1,2,3,4-butane-tétracarboxylate de tétraméthyle.

3. Procédé conforme à la revendication 1, dans lequel on effectue l'électrolyse à une vitesse suffisante pour que la quantité de 2-méthoxy-succinate de dialkyle produit par une réaction chimique concurrente soit maintenue à une valeur représentant moins de 5 % du maléate de dialkyle ayant réagi.

4. Procédé conforme à la revendication 1, dans lequel le carboxylate de métal alcalin, soluble dans l'alcanol et utilisé comme électrolyte support, est de l'acétate de sodium.

5. Procédé conforme à la revendication 2, dans lequel on sépare le 1,2,3,4-butane-tétracarboxylate de tétraméthyle d'avec le milieu d'électrolyse par refroidissement et cristallisation.

6. Procédé conforme à la revendication 2, dans lequel la concentration initiale du maléate de diméthyle dans le milieu d'électrolyse vaut au moins 15 % en poids, et l'on poursuit l'électrolyse jusqu'à ce qu'au moins environ 95 % du maléate de diméthyle ait réagi.

7. Procédé conforme à la revendication 6, dans lequel la cellule d'électrolyse est une cellule à circulation que l'on fait fonctionner à des densités de courant d'au moins 15 mA/cm².

8. Procédé conforme à la revendication 7, dans lequel les densités de courant se situent dans l'intervalle allant de 15 à 100 mA/cm².
